# EUROPEAN PATENT SPECIFICATION

(11) **EP 4 001 887 B1**
(45) Date of publication and mention of the grant of the patent: **20.08.2025**
(21) Application number: 21208632.6
(22) Date of filing: 16.11.2021
(51) Int. Cl.: G01N 1/22, B01L 7/00, B64D 11/04, B64D 13/06

(54) **METHODS OF OBTAINING A BIOLOGICAL SAMPLE REPRESENTATIVE OF A PASSENGER CABIN ON AN AIRCRAFT USING AN AIR CYCLONIC COLLECTOR VIA A CABIN EXHAUST VALVE**
VERFAHREN ZUR ENTNAHME EINER FÜR DIE PASSAGIERKABINE REPRÄSENTATIVEN BIOLOGISCHEN PROBE IN EINEM FLUGZEUG UNTER VERWENDUNG EINES LUFTZYKLONSAMMLERS ÜBER EIN KABINENAUSLASSVENTIL
PROCÉDÉS D'OBTENTION D'UN ÉCHANTILLON BIOLOGIQUE REPRÉSENTATIF DE LA CABINE DE PASSAGERS D'UN AÉRONEF À L'AIDE D'UN COLLECTEUR D'AIR CYCLONIQUE PAR L'INTERMÉDIAIRE D'UNE SOUPAPE D'ÉCHAPPEMENT DE LA CABINE

(30) Priority: 16.11.2020 US 202063114064 P; 16.11.2020 US 202063114330 P; 16.11.2020 US 202063114339 P; 16.11.2020 US 202063114350 P; 16.11.2020 US 202063114400 P; 16.11.2020 US 202063114157 P; 16.11.2020 US 202063114386 P; 16.11.2020 US 202063114366 P
(43) Date of publication of application: 25.05.2022
(73) Proprietor: B/E Aerospace Inc., Winston Salem, NC 27105 (US)
(72) Inventor: SURAWSKI, Eric, Wethersfield, 06109 (US); CRAWFORD, Kenneth, Westwood, MA, 02090 (US); ARMY, Donald E., Enfield, 06082 (US); WALSH, Kevin P., Enfield, 06082 (US); SHEA, Brian R., Windsor, 06095 (US); HO, Tony, Glastonbury, CT, 06033 (US)
(74) Representative: Dehns

(56) References cited:
- US-A1- 2004 038 385
- US-A1- 2011 159 596
- US-A1- 2014 370 519
- US-B1- 6 468 330
- US-B1- 7 390 339

## Description

### CROSS-REFERENCE TO RELATED APPLICATION

The following application claims priority to U.S. Provisional Patent Applications with the following Serial Nos. 63/114,330, 63/114,339, 63/114,350, 63/114,400, 63,114,064, 63/114,157, 63/114,386, 63/114,366 all filed on November 16, 2020; and Patent Application Serial No. 63/043,414 filed on June 24, 2020.

### BACKGROUND OF THE INVENTION

### 1. Field of the Invention

The present application is related to a method used collect a representative air sample of an aircraft, more specifically to a method for collecting a biological sample on an aircraft.

### 2. Description of Related Art

The spread progression of SARS-CoV-2 around the world has risen a red flag: Economic economic globalization creates systemic risks. As trade, finance, travel, cyber and other networks grow in scale and interact, they become more complex and unstable. The transporters of the goods of the global economy, such as major airport hubs, are also spreaders of the pathogens. The 2008 global financial crisis provided a dramatic example of how contagions could spread from the US to global markets overnight. So too has the rapid spread of cyber viruses. In health, rising life expectancy and success in preventing a repeat of the devastating influenza pandemic of 1918, which infected about one-third of the world's population and killed as many as 50m people, has created a false sense of security. But the world is now more interdependent. For example, China represents almost one-fifth of global output, is integral to global supply chains, example, China represents almost one-fifth of global output, is integral to global supply chains, and its tourists spend over $260 billion annually. The COVID-19 pandemic shed light on the need for better monitoring, detecting, and isolating ill passengers, specifically due to the havoc that was wreaked detrimental impact on the global economy, specifically air travel due to closed borders, movement restrictions, and testing requirements.

However, the COVID-19 pandemic the air travel industry has proven that air travel can be safe and that aircraft cabins have a well-managed airflow that inhibits minimize the risk for transmission of virus, and that being seated onboard an aircraft is safer than shopping in large stores. Governments and other authorities need to assume that aircraft are contaminated until proven "clean", as 25% of COVID-19 cases are asymptomatic or pre-symptomatic; but still contagious. Thus, if borders shutdown and a drastic reduction in international travel global passenger travel is greatly reduced. To date travelers and governments have relied on individual diagnostic tests. The uncertainty of the results has reduced people's inclination to travel and subsequent airline inclination to maintain routes.

Accordingly, conventional systems and methods of monitoring infections has not lived up to requirements of the fast paced modern world. Thus, there is still a need in the art for an improved on-board virus and pathogen detection system, and method of use. The present disclosure provides a solution for this need. US 2004/038385 A1 describes a system for autonomous monitoring of bioagents. US 2014/370519 A1 describes a universal sample preparation system. US 6 468 330 B1 describes a multi-cyclone bio collector and concentrator. US 7 390 339 B1 describes a vortex separator. US 2011/159596 A1 describes a substance detector.

### SUMMARY

A method of monitoring aircraft air includes removing a conical collector through a cabin exhaust valve of the aircraft wherein ambient cabin air has been driven from the cabin over a reagent fluid within the conical collector in order to collect a representative sample within the reagent fluid. Ambient cabin air can be driven by an air circulation machine. The method can include extracting and purifying the reagent fluid by passing the concentrated reagent fluid through silica columns, and by extracting and purifying by passing the concentrated reagent fluid through magnetized beads. Concentrating the reagent fluid can include using a concentration pipette. The conical collector can be removed by hand, without requiring any special tooling or machinery to access the collector, since the conical collector is open to ambient air.

These and other features of the methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below with reference to certain figures, wherein:

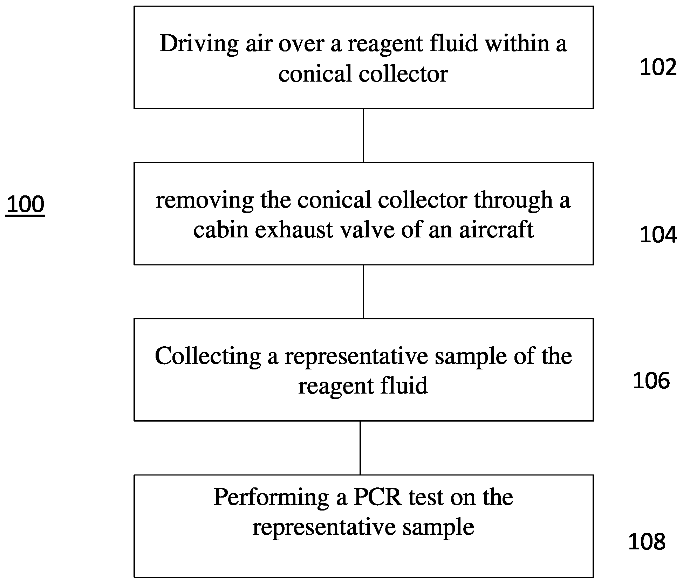

FIG. 1 is a block diagram of a method of removing a conical collector according to the disclosure.

### DETAILED DESCRIPTION OF THE PREFERRED EMBODIMENTS

Reference will now be made to the drawings wherein like reference numerals identify similar structural features or aspects of the subject disclosure. For purposes of explanation and illustration, and not limitation, a partial view of an exemplary embodiment of a method in accordance with the disclosure is shown in FIG. 1 and is designated generally by reference character 100. The methods and system described below can be used to collect a bulk sample, representative of each passenger on the aircraft and to test it to provide a bulk screening of the aircraft prior to arrival of the aircraft at a destination, and quickly turned around for a fast dispatch of the next flight.

FIG. 1 shows a method 100 for monitoring aircraft air includes removing a conical collector through a cabin exhaust valve of the aircraft wherein ambient cabin air has been driven from the cabin over a reagent fluid within the conical collector in order to collect a representative sample within the reagent fluid. Ambient cabin air can be driven 102 by an air circulation machine. The method includes extracting and purifying 104 the reagent fluid by passing the concentrated reagent fluid through silica columns, and by extracting and purifying by passing the concentrated reagent fluid through magnetized beads. Concentrating the reagent fluid can include using a concentration pipette.

The fluid can be collected at the bottom of the conical collector or be smeared on the inner surface of the conical collector. The conical collector can be removed by hand, without requiring any special tooling or machinery to access the collector. The conical collector is open to ambient air, and can be removed by hand through outflow valve.

A system not forming part of the present invention for enabling the method described above includes a HEPA filter positioned in a recirculation flow path of an aircraft configured to collect flow path air, and at least one removable strip attached to the strip for removing after each flight. The inlet of the conical collector includes a smaller diameter than the outlet of the conical collector. The conical main body can taper down away from the inlet. The particulate samples include droplets exhaled from passengers throughout a duration of a flight. The collector can be mounted within a mounting slot in the outlet flow path upstream from the outflow valve, wherein the collector is positioned within the mounting slot. The collector can be configured and adapted to be removed from a mounting slot of the collector for testing of the collected sample and a new collector to be promptly placed within the mounting slot for the next flight.

The methods and systems of the present disclosure, as described above and shown in the drawings, provide for improved collector changeover and improved data collection of air aboard an aircraft. While the apparatus and methods of the subject disclosure have been shown and described with reference to preferred embodiments, those skilled in the art will readily appreciate that changes and/or modifications may be made thereto without departing from the scope of the subject disclosure.

## Claims

1. A method comprising:
removing (104) a conical collector through a cabin exhaust valve of the aircraft wherein ambient cabin air has been driven from the cabin over a reagent fluid within the conical collector in order to collect a representative sample within the reagent fluid.

2. The method of claim 1, wherein ambient cabin air is driven by an air circulation machine (102).

3. The method of claim 1 or 2, further comprising extracting and purifying the reagent fluid including passing the concentrated reagent fluid through silica columns.

4. The method of claim 3, wherein extracting and purifying includes passing the concentrated reagent fluid through magnetized beads.

5. The method of claim 3, wherein concentrating the reagent fluid includes a concentration pipette.

6. The method of claim 3, wherein the conical collector is removed by hand.

7. The method of claim 3, wherein the conical collector is open to ambient air.

8. The method of any preceding claim, further comprising testing the representative sample.

## Patentansprüche

1. Verfahren, umfassend:
Entfernen (104) eines konischen Kollektors durch ein Kabinenauslassventil des Flugzeugs, wobei Umgebungskabinenluft aus der Kabine über ein Reagenzfluid innerhalb des konischen Kollektors geleitet wurde, um eine repräsentative Probe innerhalb des Reagenzfluids zu sammeln.

2. Verfahren nach Anspruch 1, wobei Umgebungskabinenluft durch eine Luftzirkulationsmaschine (102) geleitet wird.

3. Verfahren nach Anspruch 1 oder 2, ferner umfassend das Extrahieren und Reinigen des Reagenzfluids, beinhaltend das Leiten des konzentrierten Reagenzfluids durch Silikasäulen.

4. Verfahren nach Anspruch 3, wobei Extrahieren und Reinigen das Leiten des konzentrierten Reagenzfluids durch magnetisierte Kügelchen beinhaltet.

5. Verfahren nach Anspruch 3, wobei Konzentrieren des Reagenzfluids eine Konzentrationspipette beinhaltet.

6. Verfahren nach Anspruch 3, wobei der konische Kollektor von Hand entfernt wird.

7. Verfahren nach Anspruch 3, wobei der konische Kollektor für Umgebungsluft offen ist.

8. Verfahren nach einem der vorhergehenden Ansprüche, ferner umfassend das Testen der repräsentativen Probe.

## Revendications

1. Procédé comprenant :
le retrait (104) un collecteur conique à travers une soupape d'échappement de cabine de l'aéronef dans lequel l'air ambiant de la cabine a été entraîné de la cabine sur un fluide réactif à l'intérieur du collecteur conique afin de collecter un échantillon représentatif dans le fluide réactif.

2. Procédé selon la revendication 1, dans lequel l'air ambiant de la cabine est entraîné par une machine de circulation d'air (102).

3. Procédé selon la revendication 1 ou 2, comprenant également l'extraction et la purification du fluide réactif, y compris le passage du fluide réactif concentré à travers des colonnes de silice.

4. Procédé selon la revendication 3, dans lequel l'extraction et la purification comportent le passage du fluide réactif concentré à travers des billes magnétisées.

5. Procédé selon la revendication 3, dans lequel la concentration du fluide réactif comporte une pipette de concentration.

6. Procédé selon la revendication 3, dans lequel le collecteur conique est retiré à la main.

7. Procédé selon la revendication 3, dans lequel le collecteur conique est ouvert à l'air ambiant.

8. Procédé selon une quelconque revendication précédente, comprenant également le test de l'échantillon représentatif.
